# EUROPÄISCHE PATENTSCHRIFT

(11) **EP 1 437 994 B1**
(45) Veröffentlichungstag und Bekanntmachung des Hinweises auf die Patenterteilung: **12.04.2006**
(21) Anmeldenummer: 02781122.3
(22) Anmeldetag: 07.10.2002
(51) Int. Cl.: A61F 13/15

(54) **VERFAHREN ZUR HERSTELLUNG VON HYGIENEERZEUGNISSEN**
METHOD FOR PRODUCING HYGIENE ARTICLES
PROCEDE DE PRODUCTION D'ARTICLES D'HYGIENE

(30) Priorität: 09.10.2001 DE 10149772
(43) Veröffentlichungstag der Anmeldung: 21.07.2004
(73) Patentinhaber: Microtac Systems AG, 08525 Plauen (DE)
(72) Erfinder: KRÄMER, Walter, 53225 Bonn (DE)
(74) Vertreter: Seerig & Hübner
(86) Internationale Anmeldenummer: PCT/DE2002/003762
(87) Internationale Veröffentlichungsnummer: WO 2003/032879

(56) Entgegenhaltungen:
- WO-A-01/32403
- WO-A-98/28134

## Beschreibung

Die Erfindung betrifft ein Verfahren zur Herstellung von dem Aufbau nach fünfbahnigen Hygieneerzeugnissen, welche produktionstechnisch vorzugsweise als komplette Babywindel ausgestattet ist.

Bisher ist mit der DE 40 41 228 die Lösung der Herstellung einer Hygienefolie mit verstärkter Saugfähigkeit bekannt, bei welcher eine Zellstoffkartonbahn durch mechanische Behandlung zu einer voluminösen Fluff-Schicht aufbereitet, eine Wäscheschutzfolienbahn parallel zur Fluff-Schicht zugeführt wird und diese Bahnen gemeinsam durch Tissue-Zellstoff und Vliesstoff umhüllt werden. Diese Lösung ist dadurch gekennzeichnet, daß eine wasserhaltige Suspension eines Superabsorbers auf die Basisschicht, den Zeltstoffkarton aufgetragen wird.

Dieser Lösung haftet jedoch der Nachteil an, daß die auf solche Weise gewonnene Hygienefolie noch eine erhebliche zusätzlich nachfolgende Bearbeitung erfahren muß, um beispielsweise eine komplette, also fertige Babywindel zu erzeugen. Das betrifft insbesondere das Verkleben beziehungsweise Verschweißen von Rändem, oder aber die Ausstattung mit einem Klettverschluß, welcher separat gefertigt und nachträglich aufgebracht werden muß.

Ferner ist eine weitere Lösung bekannt, bei welcher aus einer dreibahnigen, außen atmungsaktiven Grundfolie und in der Mitte angeordneten Dichtfolie, welche an den Seiten verschweißt oder verklebt wird, miteinander verbunden werden.

Auch bei dieser Lösung wirkt nachteilig, daß die Verschlüsse als Klebe- oder auch Klettverschluß separat gefertigt und im Nachhinein aufgebracht werden müssen. Dieser nachträgliche Aufwand verteuert das Erzeugnis unnötig und steht einer guten Ökonomie im Wege.

Mit der DE 201 00 065 ist eine weitere Lösung für eine Windel mit lösbaren Verschlüssen bekannt, welche dadurch gekennzeichnet ist, daß die Windel an ihren Verschlussteilen mit Positionsmarkierungen versehen ist.

Dieser Lösung haftet der Nachteil an, daß sie außer ihren Verschlussteilen mit Markierungen keinen effektiven Aufbau aufweist, welcher durch seine Anordnung in der Lage ist, eine hohe Aufnahmefähigkeit von Flüssigkeit zu bewirken.

Schließlich ist noch die Lösung einer dreibahnigen Grundfolie bekannt, welche außen atmungsaktiv ausgestaltet ist und in der Mitte eine Dichtfolie aufweist., welche an den Stoßseiten verschweißt oder verklebt ist, wobei die Verschlüsse als Klebe- oder Klettverschlüsse separat gefertigt und aufgebracht werden.

Dieser Lösung haftet der Nachteil an, daß es sich bei der Herstellung dieses Erzeugnisses um ein sehr aufwendiges Herstellungsverfahren handelt. Es werden mindestens drei bis vier Arbeitsgänge notwendig, unabhängig von einer extrudierten Herstellung der Folie. Diese Herstellung ist sehr kostenintensiv und wirkt sich gleichermaßen verteuernd auf den Preis des Enderzeugnisses aus. Zudem ist die Entsorgung fraglich.

Die Erfindung stellt sich daher die Aufgabe ein kostengünstiges Herstellungsverfahren für Hygieneerzeugnisse in einem einzigen Zyklus zu schaffen, bei dem technologisch nach dem Extrusionsprozeß direkt, beziehungsweise inline das Endprodukt konfektioniert wird. Erfindungsgemäß soll das Herstellungsverfahren eine hohe Produktionssicherheit aufweisen, bei einem günstigen Preis-Leistungsverhältnis. Ferner soll der verwendete Grundstoff leicht und problemlos zu entsorgen sein und das Endprodukt soll zudem ein verbraucherfreundliches Handling aufweisen.
Die Aufgabe der Erfindung wird gelöst, durch die im kennzeichnenden teil des Anspruchs 1 aufgezeigten technischen Merkmale.

Das Wesen der Erfindung besteht in einem Verbund von fünf nebeneinanderliegenden Bahnen, welche in einem Extrusionsprozeß mittels zweier Extruder hergestellt werden und nachfolgend direkt, beziehungsweise inline konfektioniert werden. Die vorzugsweise Polyethylenfolie, aber auch Polypropylenfolie wird fünfbahnig mittels der beiden Extruder über eine Filterung und eine Dosierpumpe sowie einem spezifischen, mit mehreren Düsen ausgestatteten Breitschlitzwerkzeug über einem Gießwalzstuhl hergestellt. Dabei erzeugt der erste Extruder die Mittel- und die beiden Außenbahnen. Der zweite Extruder erzeugt die beiden atmungsaktiven Bahnen, welche zwischen den beiden Außenbahnen auf der Mittelbahn aufgesetzt, angeordnet sind. Der Klettverschluß des Erzeugnisses wird nachfolgend sowohl auf der Gießwalze, als auch durch ein nachgeschaltetes Prägewalzenpaar im Produktionsfluß gleichzeitig erzeugt.
Um die Atmungsaktivität zu bewirken, wird die Folie in Längsrichtung inline verstreckt. In einem getrennten Verfahren wird die atmungsaktive Folie, zusammengesetzt aus einem biologischen und einem chemischen Polymer, verfahrenstechnisch mittels Mikrowelle hergestellt. Hierbei werden die biologischen Polymere aus der extrudierten Folie mittels Mikrowelle ausgelagert. Schließlich wird noch ein Saugkissen auf der flüssigkeitsdichten Mittelbahn durch den zweiten Extruder aufgebracht und mit einer darüber befindlichen flüssigkeitsdurchlässigen Oberfolie als Deckfolie abgechlossen, welche randseitig mit einem Dehnband ausgestattet ist.
Erfindungsgemäß erfolgt auf vorbeschriebene Weise ein Compound des Polymers durch Extrudierung, wobei das eingesetzte Extrusionswerkzeug aus einer mehrkanaligen Breitschlitzdüse besteht, mit deren Hilfe der fünfbahnige Aufbau der Hygienefolie erfolgt. Schließlich wird das Ausformen eines Klettverschlusses gleichzeitig inline beim Herstellungsprozeß durch den zweiten Extruder mit durchgeführt und Kräuselfaser auf die Außenbahn als Schließelement für den Klettverschluß aufgesprüht.

Die Erfindung soll nachstehend an einem Ausführungsbeispiel näher erläutert werden. In der zugehörigen Zeichnung zeigen:

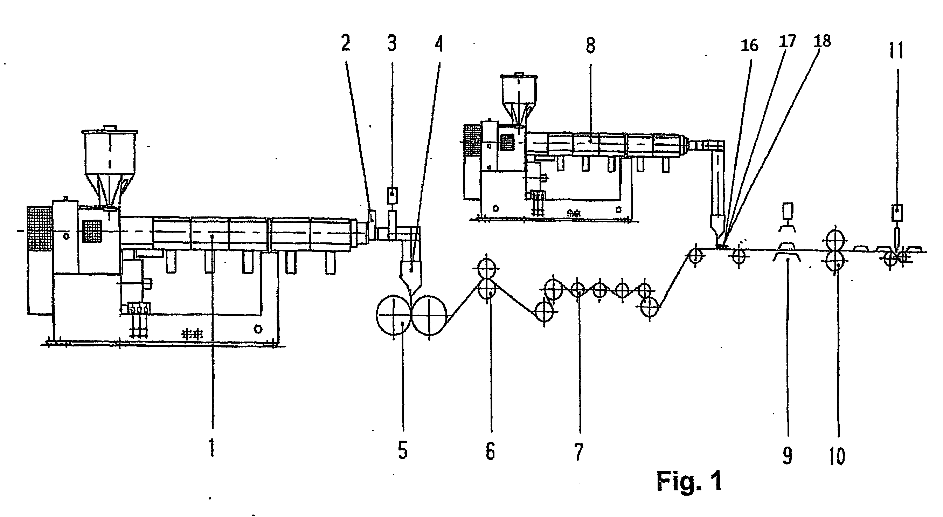

- Figur 1:: die schematische Produktionsanlage mit Ablauf

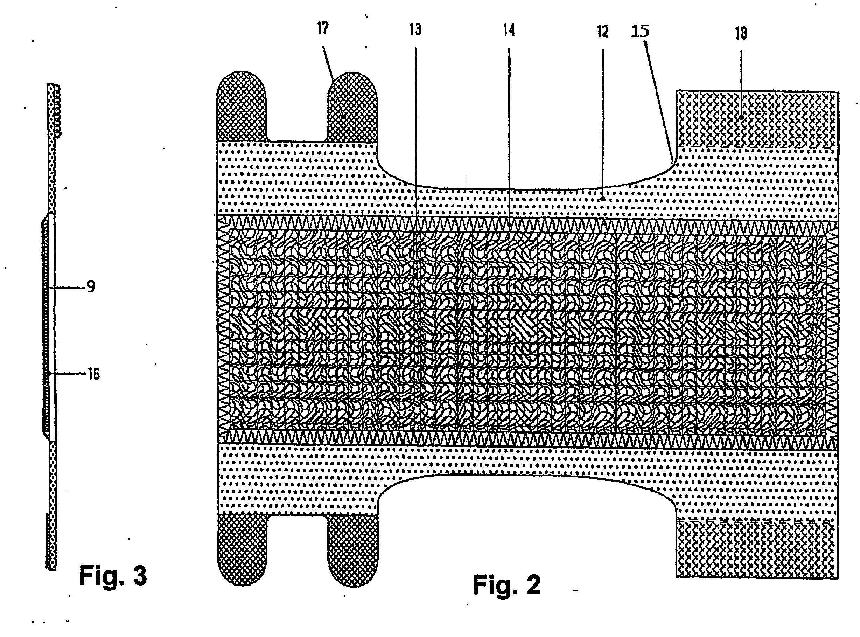

- Figur 2:: Draufsicht auf die Hygienefolie
- Figur 3:: Schnitt durch die Hygienefolie

Mit einem Extruder 1 werden eine Mittelbahn 13 und zwei Außenbahnen 12 extrudiert. Technologisch werden sämtliche fünf Kanäle eines mehrkanaligen Breitschlitzwerkzeuges 4 für diesen Prozeß genutzt. Dadurch werden auch verfahrenstechnisch sämtliche fünf Foliebahnen welche die Hygienefolie bilden, in einem Spalt extrudiert, was bei bisheriger traditioneller Fertigung mehrere Arbeitsgänge umfasste, die nunmehr komplex zusammengefasst sind. Im Extrusionsprozeß ist durch ein Sieb eine Filterung 2 angeordnet sowie nachfolgend eine Dosierpumpe 3 einbezogen, wobei das eingesetzte Vorzugsmaterial Polyethylen über das vorgenannte Breitschlitzwerkzeug 4 und ein Gießwalzenpaar 5 geführt wird. Nachfolgend durchlaufen die so extrudierten Bahnen als die Mittelbahn 13 und die beiden Außenbahnen 12 ein Formwalzenpaar 6, von wo sie in eine darauf folgende Verstreckung 7 überführt werden. Hier findet die Verstreckung der Foliebahnen in Längsrichtung inline statt, wodurch eine gute Atmungsaktivität bewirkt wird. Der Inline-Verstreckung 7 nachfolgend wird durch den zweiten Extruder 8 ein Suagkkissen 16 aufgebracht Schließlich wird auf die Hygienefolie im weiteren Produktionsprozeß eine flüssigkeitsdurchlässige Deckfolie 9 aufgebracht, welche zudem an ihrem Rand mit einem Dehnband 14 ausgestattet ist. Nachfolgend schließt sich die Station mit der Formschneidung 10 an, wobei die kurvenförmigen seitlichen Einschnitte 19 gemäß Ausführungsbeispiel für Babywindeln herausgeschnitten werden. Unmittelbar danach wird das fertige Hygieneerzeugnis in der Station Ablängung 11 auf die gewünschte Länge des Produktes geschnitten.
Wie aufgezeigt, wird gemäß dem Ausführungsbeispiel ein Hygieneerzeugnis in Form einer Babywindel komplett in einem einzigen Arbeitszyklus erzeugt, die aus den beiden atmungsaktiven Außenbahnen 12 als Breathhable-Filmbahn und einer flüssigkeitsdichten Mittelbahn 13 besteht, welche mit einer Deckfolie 9 ausgestattet ist, die randseitig mit einem Dehnband 14 versehen ist. Schließlich ist ein Saugkissen (16) mit einer flüssigkeitsdurchlässigen Deckfolie 9 ausgebildet und ein Klettverschluß 17 mit vorzugsweise zwei Klettlaschen auf dem äußeren Bereich der Außenbahnen 12 angeordnet. Die beiden Klettlaschen des Klettverschlusses 17 werden beim späteren Gebrauch sicher mit der lagegleich angeordneten Kräuselfaser 18 verbunden, wodurch ein zuverlässiges Schließen der Babywindel gewährleistet ist. Der gerundete Einschnitt 15, welcher in der Station der Formschneidung 10 bewirkt wird, dient lediglich einer guten Körperanpassung der Babywindel und zeigt die problemlose Einbindung eines derartigen Arbeitsganges in den Produktionszyklus.

### Aufstellung der verwendeten Bezugszeichen

- 1 -: Extruder
- 2 -: Filterung
- 3 -: Dosierpumpe
- 4 -: mehrkanaliges Breitschlitzwerkzeug
- 5 -: Gießwalzenpaar
- 6 -: Formwalzenpaar
- 7 -: Verstreckung inline
- 8-: Extruder
- 9 -: flüssigkeitsdurchlässige Deckfolie
- 10-: Formschneidung
- 11 -: Ablängung
- 12-: Außenbahn Breathable-Filmbahn (Polypropylen)
- 13-: Mittelbahn (flüssigkeitsdichte Bahn PE, PP)
- 14-: Dehnband
- 15-: Einschnitt
- 16-: Saugkissen
- 17-: Klettverschluss (Klettlasche; zwei Bahnen)
- 18-: Kräuselfaser

## Patentansprüche

1. Verfahren zur Herstellung von Hygieneerzeugnissen, insbesondere Babywindeln **dadurch gekennzeichnet, daß** diese in einem einzigen Produktionszyklus im Compound mittels zweier Extruder (1,8) als fünflagige Foliebahnen unter Zuhilfenahme eines mehrkanaligen Breitschlitzwerkzeuges (4) extrudiert werden, wobei die Folie in Längsrichtung gleichzeitig eine Inline-Verstreckung (7) zur erhöhten Atmungsaktivität erfährt und ein Klettverschluss (17) mit vorzugsweise zweier Laschen sowie eine zugehörige Kräuselfaser (18) auf Breathable-Filmbahnen extrudiert werden, welche die Außenbahnen (12) bilden.

2. Verfahren zur Herstellung von Hygieneerzeugnissen nach Anspruch 1 **dadurch gekennzeichnet, daß** mit dem Extruder (1) über ein Sieb als Filterung (2) und eine Dosierpumpe (3) eine Mittelbahn (13) als flüssigkeitsdichte Bahn sowie zwei atmungsaktive Breathable-Filmbahnen als Außenbahnen (12) extrudiert werden.

3. Verfahren zur Herstellung von Hygieneerzeugnissen nach Anspruch 1 **dadurch gekennzeichnet, daß** das Ausformen eines Klettverschluses (17) mit der zugehörigen Kräuselfaser (18) durch den Extruder (8) auf die beiden Außenbahnen (12) mit vorzugsweise zwei Laschen erfolgt.

4. Verfahren zur Herstellung von Hygieneerzeugnissen nach Anspruch 1 **dadurch gekennzeichnet, daß** auf die flüssigkeitsdichte Mittelbahn (13) mittels Extruder (8) ein Saugkissen (16) aufgebracht wird, und dieses mit einer flüssigkeitsdurchlässigen Deckfolie (9) nach oben hin abschließt und randseitig mit einem Dehnband (14) ausgestattet ist.

5. Verfahren zur Herstellung von Hygieneerzeugnissen nach Anspruch 1 **dadurch gekennzeichnet, daß** dieses in einer Station Formschneidung (10) mit einem Einschnitt (15) versehen wird, welcher einer zweckdienlichen Körperanpassung dient und abschließend in der Station Ablängung (11) das Hygieneerzeugnis auf die konfektionierte Länge geschnitten wird.

6. Verfahren zur Herstellung von Hygieneerzeugnissen nach Anspruch 1 **dadurch gekennzeichnet, daß** sämtliche fünf Foliebahnen in einem Prozeß mittels des mehrkanaligen Breitschlitzwerkzeuges (4) extrudiert werden, über ein Gießwalzenpaar (5) geführt werden und nachfolgend einem Formenwalzenpaar (6) zur Formung der Folien zugeführt werden sowie anschließend die Inline-Verstreckung (7) erfolgt mit nachfolgender Aufbringung der flüsigkeitsdurchlässigen Deckfolie (9) und ihren randseitigen Dehnbändem (14).

## Claims

1. Method for producing hygiene articles, in particular baby nappies, **characterized in that** these are extruded in a single production cycle in a compound structure by means of two extruders (1, 8) as five-ply sheet webs, with the aid of a multiduct sheet die (4), the sheet simultaneously undergoing in a longitudinal direction an inline drafting (7) for the increased breathability, and a touch-and-close fastening (17) with preferably two tongues and also an associated curling fibre (18) being extruded onto breathable film webs which form the outer webs (12).

2. Method for producing hygiene articles according to Claim 1, **characterized in that**, by means of the extruder (1), a middle web (13) as a liquid-tight web and two breathable film webs as outer webs (12) are extruded via a screen as filtration (2) and via a metering pump (3).

3. Method for producing hygiene articles according to Claim 1, **characterized in that** the formation of a touch-and-close fastening (17) together with the associated curling fibre (18) takes place by means of the extruder (8) on the two outer webs (12) with preferably two tongues.

4. Method for producing hygiene articles according to Claim 1, **characterized in that** an absorbent pad (16) is applied to the liquid-tight middle web (13) by means of the extruder (8), and said absorbent pad is closed off upwardly by means of a liquid-permeable covering sheet (9) and is equipped along the edge with a stretchable tape (14).

5. Method for producing hygiene articles according to Claim 1, **characterized in that** said hygiene article is provided in a form-cutting station (10) with an incision (15) which serves for expedient body fitting, and, finally, the hygiene article is cut to the made-up length in the cutting-to-length station (11).

6. Method for producing hygiene articles according to Claim 1, **characterized in that** all five sheet webs are extruded in one process by means of the multiduct sheet die (4), are led via a pair of casting rollers (5) and are subsequently delivered to a pair of forming rollers (6) for forming the sheets, and, thereafter, the inline drafting (7) takes place, with a subsequent application of the liquid-permeable covering sheet (9) and its edge-placed stretchable tapes (14).

## Revendications

1. Procédé de production d'articles d'hygiène, notamment de couches pour bébé, **caractérisé en ce que** ceux-ci sont extrudés dans un cycle de production unique dans une structure composée au moyen de deux extrudeuses (1, 8) sous forme de bandes de feuille à cinq couches en utilisant un outil à fente large à plusieurs canaux (4), la feuille subissant dans la direction longitudinale simultanément un étirement en ligne en vue d'augmenter sa respirabilité, et une fermeture de type velcro (17) avec de préférence deux pattes ainsi qu'une fibre à frisure associée (18) étant extrudées sur des bandes de film respirable qui forment les bandes extérieures (12).

2. Procédé de production d'articles d'hygiène selon la revendication 1, **caractérisé en ce qu'**une bande centrale (13), la bande étanche aux liquides, ainsi que deux bandes de film respirable favorisant la respiration sont extrudées sous forme de bandes extérieures (12) avec l'extrudeuse (1) par le biais d'un tamis servant de filtrage (2) et d'une pompe de dosage (3).

3. Procédé de production d'articles d'hygiène selon la revendication 1, **caractérisé en ce que** la mise en forme d'une fermeture de type velcro (17) avec les fibres à frisure associées (18) s'effectue à travers l'extrudeuse (1) sur les deux bandes extérieures (12) avec de préférence deux pattes.

4. Procédé de production d'articles d'hygiène selon la revendication 1, **caractérisé en ce que** l'on applique sur la bande centrale étanche aux liquides (13), au moyen de l'extrudeuse (8) un coussin aspirant (16) et celui-ci est fermé vers le haut par une feuille de recouvrement perméable aux liquides (9) et est muni du côté des bords d'une bande extensible (14).

5. Procédé de production d'articles d'hygiène selon la revendication 1, **caractérisé en ce que** celui-ci est pourvu d'une entaille (15) dans un poste de découpe en forme (10), laquelle entaille sert d'adaptation judicieuse au corps et qu'ensuite le produit d'hygiène est découpé à la longueur de confection dans le poste de découpe à longueur (11).

6. Procédé de production d'articles d'hygiène selon la revendication 1, **caractérisé en ce que** toutes les cinq bandes de feuille sont extrudées en un processus au moyen de l'outil à fente large à plusieurs canaux (4), sont guidées sur une paire de rouleaux de coulée (5) puis sont acheminées à une paire de rouleaux de formage (6) pour la mise en forme des feuilles, et ensuite l'étirement en ligne (7) est effectué avec application subséquente de la feuille de recouvrement perméable aux liquides (6) et de ses bandes extensibles du côté des bords (14).
